# EUROPEAN PATENT SPECIFICATION

(11) **EP 3 013 316 B1**
(45) Date of publication and mention of the grant of the patent: **30.05.2018**
(21) Application number: 14732865.2
(22) Date of filing: 20.06.2014
(51) Int. Cl.: A61K 9/00, A61K 47/02, A61K 47/18, A61K 47/22, A61K 47/26, A61K 9/08, A61K 9/19, A61K 31/40, A61P 35/00

(54) **STABLE INTRAVENOUS FORMULATION**
INTRAVENÖSE STABILE FORMULIERUNG
PRÉPARATION INTRAVEINEUSE STABLE

(30) Priority: 24.06.2013 US 201361838642 P; 28.06.2013 US 201361840930 P
(43) Date of publication of application: 04.05.2016
(73) Proprietor: F. Hoffmann-La Roche AG, 4070 Basel (CH)
(72) Inventor: GALASSO, Anthony, N., Nutley, NJ 07110 (US); INBAR, Petra, Glen Rock, NJ 07452 (US); QURESHI, Farooq, West Orange, NJ 07052 (US); SAMPAT, Harendra, R., Wayne, NJ 07470 (US); ZHAN, Shangdong, North Caldwell, NJ 07006 (US)
(74) Representative: Beyermann, Jochen Carl
(86) International application number: PCT/EP2014/062982
(87) International publication number: WO 2014/206866

(56) References cited:
- WO-A1-2011/098398
- WO-A1-2013/135648

## Description

### BACKGROUND OF THE INVENTION

4-{[(2R, 3S, 5S)-4-(4-chloro-2-fluoro-phenyl)-3-(3-chloro-2-fluoro-phenyl)-4-cyano-5-(2,2-dimethyl-propyl)-pyrrolidine-2-carbonyl]-amino}-3- methoxy -benzoic Acid 1-[2-(2-methoxy-ethoxy)-ethoxycarbonyloxy]-ethyl ester (Compound A) having the formula 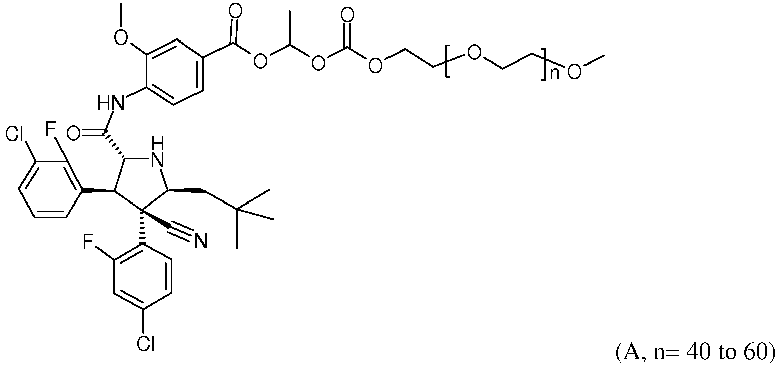 is a water soluble prodrug of 4-{[(2R,3S,4R,5S)-3-(3-chloro-2-fluoro-phenyl)-4-(4-chloro-2-fluoro-phenyl)- 4-cyano-5-(2,2-dimethyl-propyl)-pyrrolidine-2-carbonyl]-amino}-3-methoxybenzoic acid (base compound) which is a pharmacologically active MDM2 inhibitor. The base compound, as e.g. disclosed in WO 2011/098398, is a practically water insoluble compound and does not lend itself towards the development of a viable intravenous injection formulation. Compound A is obtained by covalently conjugating the base compound with a PEG (Polyethylene glycol, 2000 ± 500 Da) polymer to yield a prodrug that is relatively more soluble in water. Preferably compound A has n=44, 45, 46, 47, 48, 49, 50, 51, 52, 53, 54 and/or 55. Most preferred, n=50.

Early formulation development of Compound A for preclinical studies with normal saline and other physiologically acceptable buffered solutions demonstrate that a viable solution formulation is not an option for a commercial drug product from physico-chemical stability point of view. This is attributed to the fact that Compound A hydrolyzes in aqueous solutions following first-order kinetics to form the base compound as the major degradation product. The most stable pH range is around 3-5 from stability perspective for Compound A. The degradation rate for Compound A increases about 2-5 times with every 10 °C increase in temperature. The compound is also vulnerable to oxidation leading to the formation of the base compound as the major oxidation product. Compound A is also light sensitive leading to the formation of the base compound and other degradants. Even tiny amounts of the base compound as a degradation product leads to a rapid loss of product shelf life through particulate formation (precipitation) and gelation thus rendering the product unsuitable for patient administration. Consequently, it is an object of the present invention to provide stable formulations for intravenous administration of Compound A.

### SUMMARY OF THE INVENTION

Compound A has been developed as a stable lyophilized formulation for intravenous administration. Alternatively, Compound A may be formulated in solution and stored as a frozen solution (-20°) prior to intravenous administration. The intravenous route of administration of Compound A offers higher exposures of its base compound with potentially lower PK variability and also controls overdosing by stopping the fluid flow of drug substance through the intravenous line.

### DETAILED DESCRIPTION OF THE INVENTION

The following formulation composition was developed to provide better drug product performance and shelf life stability. If not explicitly otherwise indicated, the amounts indicated below are in relation to a final reconstitution volume of 1ml, as e.g. also indicated in the accompanying working examples.

The present invention comprises from about 0.1 mg to about 100 mg of Compound A, preferably where Compound A has n=40 to 60, from about 10mM to about 100mM of a buffering agent, from about 25 mg to about 125 mg of a lyophilization bulking agent and an isotonicity builder. The resultant formulation should have a pH of about 5-7 via adjustment with HCl or NaOH. The final reconstitution volume is 1ml.

A further aspect of the invention comprises from about 1 mg to 100 mg of Compound A wherein n=40 to 60, from about 10mM to about 50 mM of a buffering agent and from about 50 mg to about 100 mg of a lyophilization bulking agent.

In a further aspect of the invention Compound A wherein n=40-60 is present as about 30 to 75 mg of the formulation.

In a further aspect of the invention Compound A wherein n=40-60 is present as about 50 to 75 mg of the formulation.

In a further aspect of the invention Compound A wherein n=40-60 is present as about 40 to 50 mg of the formulation, preferably 41, 42, 43, 44, 45, 46, 47, or 48 mg of Compound A in a reconstitution volume of 1ml.

In a further aspect of the invention Compound A whrein n=40-60 is present as about 50mg of the formulation.

In a further aspect of the invention Compound A wherein n=44, 45, 56, 47, 48, 49, 50, 51, 52, 53, 54 and/or 55 comprises about 0.1 mg to about 100 mg in the formulations of the present invention, more preferably, about 1 mg to about 100 mg, more preferably about 30 mg of the formulation, and about 75 mg and about 50 mg of the formulation

In a further aspect of the invention the bulking agent is Trehalose, preferably Trehalose dehydrate, and is present as about 50 mg to about 100 mg, preferably about 75 to about 95 mg, of the formulation.

In a further aspect of the invention the bulking agent is Dextrose and is present as about 30 mg to about 75 mg, preferably about 40 to about 60 mg, of the formulation.

In a further aspect of the invention the bulking agent is Mannitol and is present as about 25 mg to about 75 mg, preferably about 30 to about 60 mg, of the formulation.

In a further aspect of the invention the bulking agent is Sucrose and is present as about 70 mg to about 110 mg, preferably about 75 to about 100 mg, of the formulation.

In a further aspect of the invention the bulking agent is Lactose and is present as about 70 mg to about 120 mg, preferably about 90 to about 110 mg, of the formulation.

In a further aspect of the invention the buffering agent is present as about 10mM to about 100 mM, preferably about 10 mM to about 50 mM, of the formulation.

The term "buffering agent" as used herein denotes a pharmaceutically acceptable excipient, which stabilizes the pH of a pharmaceutical preparation. Suitable buffers are well known in the art and can be found in the literature. Preferred pharmaceutically acceptable buffers comprise but are not limited to histidine-buffers, citrate-buffers, succinate-buffers, acetate-buffers and phosphate-buffers, especially, Succinic acid (20-50 mM) and Phosphoric acid (10-50 mM). Most preferred buffers comprise citrate, L-histidine or mixtures of L-histidine and L-histidine hydrochloride. Other preferred buffer is acetate buffer. Independently from the buffer used, the pH can be adjusted with an acid or a base known in the art, e.g. hydrochloric acid, acetic acid, phosphoric acid, sulfuric acid and citric acid, sodium hydroxide and potassium hydroxide.

The preferred" bulking agent" is amorphous trehalose, but trehalose dihydrate, lactose, sucrose, sorbitol, glucose, raffinose , mannitol, dextran and lower molecular weight amino acids such as glycine, valine and arginine etc. and other bulking agents known to the person of skill in the art may also be utilized.

As diluents for the formulated solution or reconstituted solution from the lyophilized powder the following diluents such as sodium chloride 0.9% Sodium, 5% Dextrose, water for injection, Lactated Ringers solution or half normal saline may also be used. It is to be appreciated that the bulking agent may also act as the isotonicity building agent.

In one embodiment, the present invention comprises a pharmaceutical lyophilized formulation comprising about 50 mg of 4-{[(2R, 3S, 5S)-4-(4-chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy -benzoic Acid 1-[2-(2-methoxy-ethoxy)-ethoxycarbonyloxy]-ethyl ester of the formula 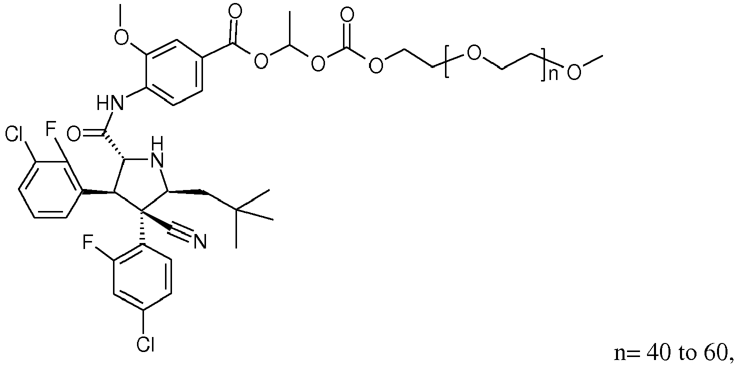 about 3.1 mg of Histidine , about 85 mg of a Trehalose dehydrate and an isotonicity builder, said formulation having a pH of from about 5 to about 7 in a final reconstitution volume of 1ml.

The present invention further comprises the above pharmaceutical lyophilized formulation wherein n is 44, 45, 46, 47, 48, 49, 50, 51, 52, 53 or 55.

The present invention further comprises the above pharmaceutical lyophilized formulation of claim 25 wherein n=50.

The present invention also comprises a pharmaceutical lyophilized formulation comprising about 435.83 mg of 4-{[(2R, 3S, 5S)-4-(4-chloro-2-fluoro-phenyl)-3-(3-chloro-2-fluoro-phenyl)-4-cyano-5-(2,2-dimethyl-propyl)-pyrrolidine-2-carbonyl]-amino}-3- methoxy - benzoic Acid 1-[2-(2-methoxy-ethoxy)-ethoxycarbonyloxy]-ethyl ester of the formula 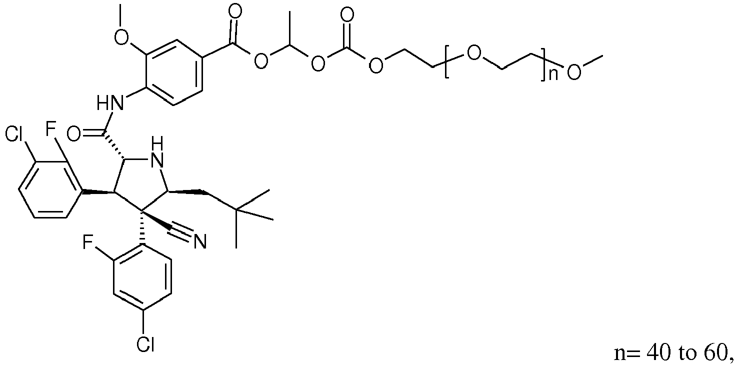 about 14.77 mg of L-Histidine, about 2.196 mg of L-Histidine HCl Monohydrate, about 756.70 mg of Trehalose dehydrate and an isotonicity builder to give a final volume of 10ml, said formulation having a pH of from about 5 to about 7.

Within this embodiment n is preferably selected from 44, 45, 46, 47, 48, 49, 50, 51, 52, 53 and/or 55.

The present invention further comprises the above pharmaceutical lyophilized formulation wherein n=50.

The present invention may be exemplified by various formulations as shown in the Examples below, which illustrates the invention without limitation.

### EXAMPLES

### Example 1

| Ingredient | Amount per mL |
|---|---|
| Compound A | 30 mg |
| Histidine USP (buffer) | 3.1 mg |
| Trehalose Dihydrate | 85 mg |
| HCl/N aOH | q.s. to pH 6 |
| Water for Injection | q.s. to 1 mL |

### Example 2

| Ingredient | Amount per mL |
|---|---|
| Compound A | 30 mg |
| Histidine USP (buffer) | 3.1 mg |
| Sodium Chloride | 9 mg |
| HCl/N aOH | q.s. to pH 6 |
| Water for Injection | q.s. to 1 mL |

### Example 3

| Ingredient | Amount per mL |
|---|---|
| Compound A | 30 mg |
| Histidine | 3.1 mg |
| Dextrose | 50 mg |
| HCl/N aOH | q.s. to pH 6 |
| Water for Injection | q.s. to 1 mL |

### Example 4

| Ingredient | Amount per mL |
|---|---|
| Compound A | 435.83 mg |
| L-Histidine | 14.77 mg |
| L-Histidine HCl Monohydrate | 2.196 mg |
| Trehalose Dihydrate | 756.70 mg |
| Water for Injection | q.s. to 10 mL |

The solution formulations of Examples 1-4 can be compounded in the following sequence on a manufacturing scale for prepare an injectable solution and lyophilized powder.

### Sterilized Solution Procedure

1. Dissolve the buffering agent in Water for Injection and adjust the pH of the solution to target pH 6 (range 5-7)
2. Add and dissolve the bulking agent/ isotonicity building agent
3. Add and dissolve Compound A
4. Adjust the final volume of the solution to the desired batch size
5. Aseptically sterile filter the solution into a previously washed and sterilized receiving vessel using a previously washed and sterilized filter membrane/cartridge (0.1-0.22 micron).
6. The sterile filtered solution must be filled aseptically into previously washed and sterilized Type I glass vials (1 mL to 100 mL) under a class 100 facility suitable for aseptic processing.
7. Completely stopper the vials aseptically using a previously washed and sterilized serum stoppers suitable for animal/human use products.
8. Put the aluminum crimps onto the filled vials and inspect the vials for any particulates and reject the filled vials with poor quality attributes for particulate matter and also cosmetic defects.
9. Label the drug product vials with appropriate labels.
10. The above solution can be infused as is or further diluted with normal saline to achieve the desired target concentration and then infused to the subject using conventional infusion apparatus available commercially.

### Lyophilized Powder Procedure

The following procedure can be followed to make the sterile lyophilized powder for injection by following similar steps as the above solution formulation first followed by the lyophilization process to eliminate any residual water from the formulation. This will render the end product as a sterile lyophilized powder which has to be reconstituted with sterile water for injection prior to dilution with the appropriate diluents.
1. Dissolve the known amount of buffering agent in Water for Injection and adjust the pH of the solution to target pH 6 (range 5-7)
2. Add and dissolve the bulking agent and isotonicity building agent
3. Add and dissolve Compound A
4. Adjust the final volume of the solution to the desired batch size
5. Aseptically sterile filter the solution into a previously washed and sterilized receiving vessel using a previously washed and sterilized filter membrane/cartridge (0.1-0.22 micron).
6. The sterile filtered solution must be filled (desired volume per vial such as 1 mL to 3 mL in a 5 mL vial with 20 mm neck size dimension; 1 mL to 14 mL in a 20 mL vial with 20 mm neck size dimension) aseptically into previously washed and sterilized Type I glass vials under a class 100 facility suitable for aseptic processing.
7. Partially stopper the vials aseptically using a previously washed and sterilized Lyo stoppers suitable for Lyophilization and suitable animal/human use drug product.
8. Load the partially stoppered vials into the lyophilizer chamber aseptically and adjust the following lyophilizer processing condition to enable the Lyophilization step

| Step | 1 | 2 | 3 | 4 | 5 | 6 |
|---|---|---|---|---|---|---|
| Shelf Temperature °C | 5 | -40 | -30 | -15 (-20 to -5) | 15 (5 to 20) | 15 (5 to 20) |
| Ramp Rate °C/min | 0.5 | 0.5 | -- | 0.5 | 0.5 | 0.5 |

### Example 5

| Ingredient | Amount per mL |
|---|---|
| Compound A | 30 mg |
| Histidine USP (buffer) | 3.1 mg |
| Mannitol | 50 mg |
| HCl/N aOH | q.s. to pH 6 |
| Water for Injection | q.s. to 1 mL |

The formulation is prepared following the steps set forth in Examples 1-4 for the injectable solution and the lyophilized formulation.

### Example 6

| Ingredient | Amount per mL |
|---|---|
| Compound A | 30 mg |
| Histidine | 3.1 mg |
| Sucrose | 90 mg |
| HCl/N aOH | q.s. to pH 6 |
| Water for Injection | q.s. to 1 mL |

The formulation is prepared following the steps set forth in Examples 1-4 for the injectable solution and the lyophilized formulation.

### Example 7

| Ingredient | Amount per mL |
|---|---|
| Compound A | 30 mg |
| Histidine | 3.1 mg |
| Lactose | 100 mg |
| HCl/NaOH | q.s. to pH 6 |
| Water for Injection | q.s. to 1 mL |

The formulation is prepared following the steps set forth in Examples 1-4 for the injectable solution and the lyophilized formulation.

### Example 8

| Ingredient | Amount per mL |
|---|---|
| Compound A | 50 mg |
| Histidine USP (buffer) | 3.1 mg |
| Trehalose Dihydrate | 85 mg |
| HCl/N aOH | q.s. to pH 6 |
| Water for Injection | q.s. to 1 mL |

The formulation is prepared following the steps set forth in Examples 1-4 for the injectable solution and the lyophilized formulation.

## Claims

1. A pharmaceutical formulation which comprises from about 0.1 mg to about 100 mg of 4-{[(2R, 3S, 5S)-4-(4-chloro-2-fluoro-phenyl)-3-(3-chloro-2-fluoro-phenyl)-4-cyano-5-(2,2-dimethyl-propyl)-pyrrolidine-2-carbonyl]-amino}-3- methoxy -benzoic Acid 1-[2-(2-methoxy-ethoxy)-ethoxycarbonyloxy]-ethyl ester of Compound A 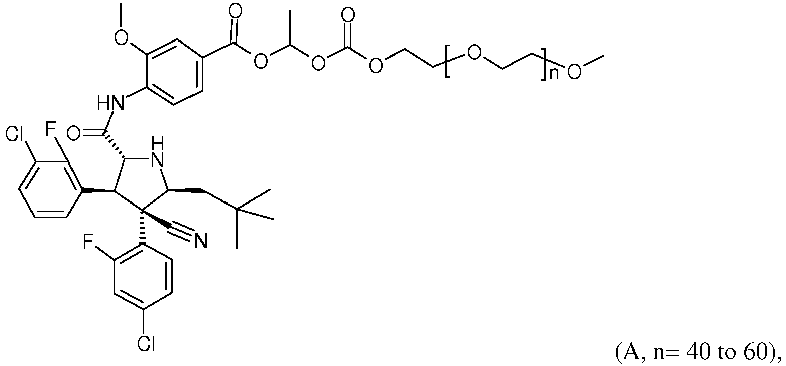 from about 10mM to about 100mM of a buffering agent, from about 25 mg to about 125 mg of a lyophilization bulking agent and an isotonicity builder having a pH of from about 5 to about 7, in a final reconstitution volume of 1ml.

2. The formulation of claim 1 wherein n is 44, 45, 46, 47, 48, 49, 50, 51, 52, 53, 54 or 55.

3. The formulation of claim 1 wherein n is 50 and Compound A is present as about 50 to about 75 mg of the formulation.

4. The formulation of claim 1 wherein Compound A is present as about 30 to about 75 mg of the formulation.

5. The formulation of claim 4 wherein Compound A is n is 44, 45, 46, 47, 48, 49, 50, 51, 52, 53, 54 or 55.

6. The formulation of claim 1 wherein Compound A is present as about 50 to about 75 mg of the formulation.

7. The formulation of claim 2 wherein Compound A is present as about 50 to about 75 mg of the formulation.

8. The formulation of claim 2 wherein Compound A is present as about 50mg of the formulation.

9. The formulation of claim 3 wherein Compound A is present as about 30 to about 75 mg of the formulation.

10. The formulation of claim 3 wherein Compound A is present as about 50mg of the formulation.

11. The formulation of claim 1 wherein the buffering agent is present as about 10mM to about 50 mM of the formulation.

12. The formulation of claim 1 wherein the bulking agent is amorphous trehalose and is present as about 75 to about 95 mg of the formulation.

13. The formulation of claim 1 wherein the bulking agent is Dextrose and is present as about 30 mg to about 75 mg of the formulation.

14. The formulation of claim 13 wherein the Dextrose is present as about 40 to about 60 mg of the formulation.

15. The formulation of claim 1 wherein the bulking agent is Mannitol and is present as about 25 mg to about 75 mg of the formulation.

16. The formulation of claim 15 wherein the Mannitol is present as about 30 to about 60 mg of the formulation.

17. The formulation of claim 1 wherein the bulking agent is Sucrose and is present as about 70 mg to about 110 mg of the formulation.

18. The formulation of claim 17 wherein the Sucrose is present as about 75 to about 100 mg of the formulation.

19. The formulation of claim 1 wherein the bulking agent is Lactose and is present as about 70 mg to about 120 mg of the formulation.

20. The formulation of claim 13 wherein the Lactose is present as about 90 to about 110 mg of the formulation.

21. The formulation of claim 1 wherein the buffering agent is Histidine and is present as about 10mM to about 100 mM of the formulation.

22. The formulation of claim 21 wherein the Histidine is present as about 10 mM to about 50 mM of the formulation.

23. The formulation of claim 1 wherein the bulking agent is present as about 50 mg to about 100 mg of the formulation.

24. A pharmaceutical lyophilized formulation comprising about 50 mg of 4-{ [(2R, 3S, 5S)-4-(4-chloro-2-fluoro-phenyl)-3-(3-chloro-2-fluoro-phenyl)-4-cyano-5-(2,2-dimethyl-propyl)-pyrrolidine-2-carbonyl]-amino}-3- methoxy -benzoic Acid 1-[2-(2-methoxy-ethoxy)-ethoxycarbonyloxy]-ethyl ester (Compound A) 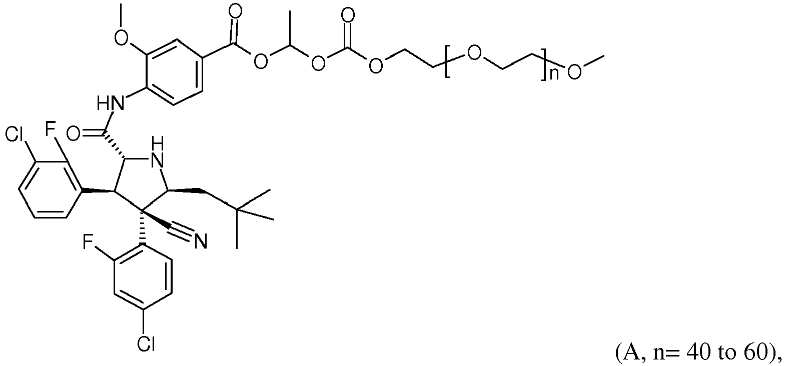 about 3.1 mg of Histidine , about 85 mg of a Trehalose dehydrate and an isotonicity builder, said formulation having a pH of from about 5 to about 7, in a final reconstitution volume of about 1ml.

25. The pharmaceutical lyophilized formulation of claim 24 wherein n is 44, 45, 46, 47, 48, 49, 50, 51, 52, 53 or 55.

26. The pharmaceutical lyophilized formulation of claim 25 wherein n=50.

## Patentansprüche

1. Eine Pharmazeutische Formulierung, umfassend von etwa 0,1 mg bis etwa 100 mg 4-{[(2R, 3S, 5S)-4-(4-Chlor-2-fluorphenyl)-3-(3-chlor-2-fluorphenyl)-4-cyano-5-(2,2-dimethylpropyl)-pyrrolidin-2-carbonyl]-amino}-3-methoxybenzoesäure-1-[2-(2-methoxyethoxy)-ethoxycarbonyloxy]-ethylester aus Verbindung A 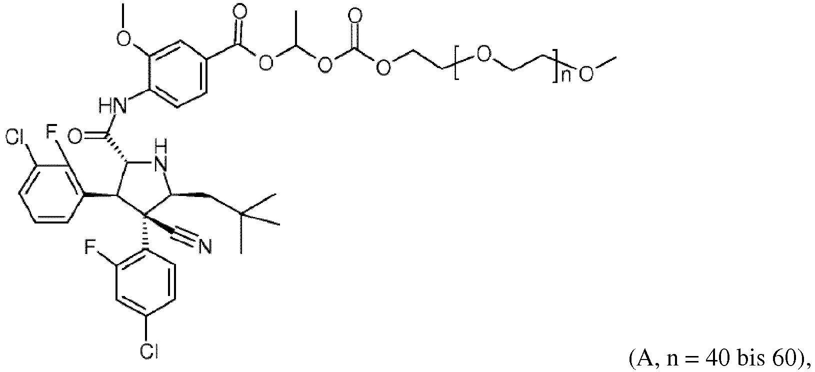 von etwa 10 mM bis etwa 100 mM einer Puffersubstanz, von etwa 25 mg bis etwa 125 mg eines Füllstoffs für Lyophilisation und eines Isotonie-Aufbaustoffs mit einem pH-Wert von etwa 5 bis etwa 7 in einem endgültigen Rekonstitutionsvolumen von 1 ml.

2. Die Formulierung nach Anspruch 1, wobei n 44, 45, 46, 47, 48, 49, 50, 51, 52, 53, 54 oder 55 ist.

3. Die Formulierung nach Anspruch 1, wobei n 50 ist und Verbindung A als etwa 50 bis etwa 75 mg der Formulierung vorhanden ist.

4. Die Formulierung nach Anspruch 1, wobei Verbindung A als etwa 30 bis etwa 75 mg der Formulierung vorhanden ist.

5. Die Formulierung nach Anspruch 4, wobei in der Verbindung A n 44, 45, 46, 47, 48, 49, 50, 51, 52, 53, 54 oder 55 ist.

6. Die Formulierung nach Anspruch 1, wobei Verbindung A als etwa 50 bis etwa 75 mg der Formulierung vorhanden ist.

7. Die Formulierung nach Anspruch 2, wobei Verbindung A als etwa 50 bis etwa 75 mg der Formulierung vorhanden ist.

8. Die Formulierung nach Anspruch 2, wobei Verbindung A als etwa 50 mg der Formulierung vorhanden ist.

9. Die Formulierung nach Anspruch 3, wobei Verbindung A als etwa 30 bis etwa 75 mg der Formulierung vorhanden ist.

10. Die Formulierung nach Anspruch 3, wobei Verbindung A als etwa 50 mg der Formulierung vorhanden ist.

11. Die Formulierung nach Anspruch 1, wobei die Puffersubstanz als etwa 10 mM bis etwa 50 mM der Formulierung vorhanden ist.

12. Die Formulierung nach Anspruch 1, wobei der Füllstoff amorphe Trehalose ist und als etwa 75 bis etwa 95 mg der Formulierung vorhanden ist.

13. Die Formulierung nach Anspruch 1, wobei der Füllstoff Dextrose ist und als etwa 30 mg bis etwa 75 mg der Formulierung vorhanden ist.

14. Die Formulierung nach Anspruch 13, wobei Dextrose als etwa 40 bis etwa 60 mg der Formulierung vorhanden ist.

15. Die Formulierung nach Anspruch 1, wobei der Füllstoff Mannit ist und als etwa 25 mg bis etwa 75 mg der Formulierung vorhanden ist.

16. Die Formulierung nach Anspruch 15, wobei Mannit als etwa 30 bis etwa 60 mg der Formulierung vorhanden ist.

17. Die Formulierung nach Anspruch 1, wobei der Füllstoff Saccharose ist und als etwa 70 mg bis etwa 110 mg der Formulierung vorhanden ist.

18. Die Formulierung nach Anspruch 17, wobei Saccharose als etwa 75 bis etwa 100 mg der Formulierung vorhanden ist.

19. Die Formulierung nach Anspruch 1, wobei der Füllstoff Lactose ist und als etwa 70 mg bis etwa 120 mg der Formulierung vorhanden ist.

20. Die Formulierung nach Anspruch 13, wobei Lactose als etwa 90 bis etwa 110 mg der Formulierung vorhanden ist.

21. Die Formulierung nach Anspruch 1, wobei die Puffersubstanz Histidin ist und als etwa 10 mM bis etwa 100 mM der Formulierung vorhanden ist.

22. Die Formulierung nach Anspruch 21, wobei Histidin als etwa 10 mM bis etwa 50 mM der Formulierung vorhanden ist.

23. Die Formulierung nach Anspruch 1, wobei der Füllstoff als etwa 50 mg bis etwa 100 mg der Formulierung vorhanden ist.

24. Eine Pharmazeutische lyophilisierte Formulierung, umfassend etwa 50 mg 4-{[(2R, 3S, 5S)-4-(4-Chlor-2-fluorphenyl)-3-(3-chlor-2-fluorphenyl)-4-cyano-5-(2,2-dimethylpropyl)-pyrrolidin-2-carbonyl]-amino}-3-methoxybenzoesäure-1-[2-(2-methoxyethoxy)-ethoxycarbonyloxy]-ethylester (Verbindung A) 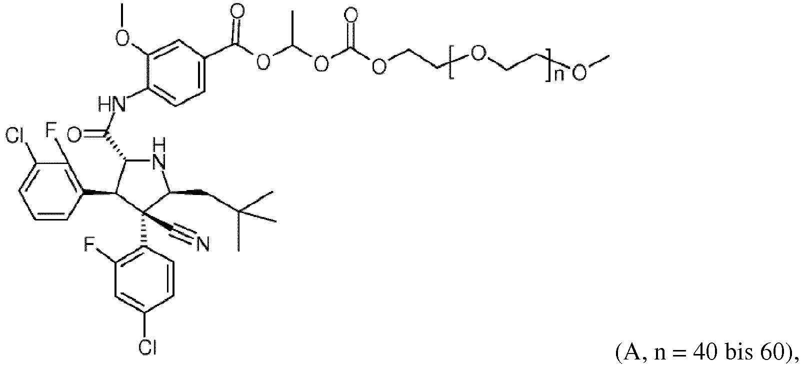 etwa 3,1 mg Histidin, etwa 85 mg eines Trehalose-Dihydrats und eines Isotonie-Aufbaustoffs, wobei die Formulierung einen pH-Wert von etwa 5 bis etwa 7 aufweist, in einem endgültigen Rekonstitutionsvolumen von etwa 1 ml.

25. Die Pharmazeutische lyophilisierte Formulierung nach Anspruch 24, wobei n 44, 45, 46, 47, 48, 49, 50, 51, 52, 53 oder 55 ist.

26. Die Pharmazeutische lyophilisierte Formulierung nach Anspruch 25, wobei n = 50.

## Revendications

1. Une préparation pharmaceutique qui comprend d'environ 0,1 mg à environ 100 mg de l'ester 1-[2-(2-méthoxy-éthoxy)-éthoxycarbonyloxy]-éthylique de l'acide 4-{[(2R, 3S, 5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque de composé A 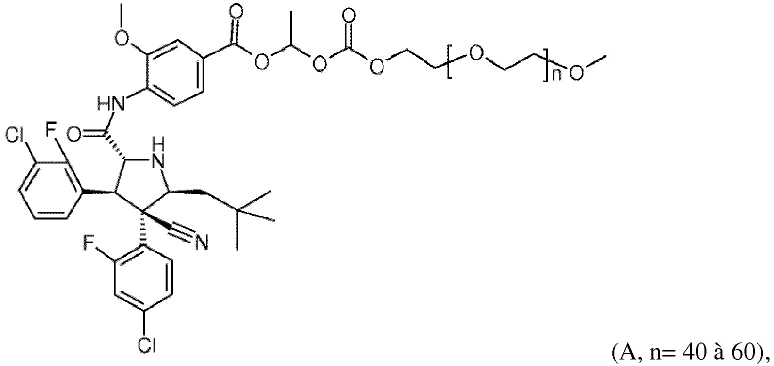 d'environ 10 mM à environ 100 mM d'un agent tampon, d'environ 25 mg à environ 125 mg d'un agent gonflant de lyophilisation et d'un adjuvant d'isotonicité ayant un pH d'environ 5 à environ 7, dans un volume de reconstitution final de 1 ml.

2. La préparation selon la revendication 1, dans laquelle n est égal à 44, 45, 46, 47, 48, 49, 50, 51, 52, 53, 54 ou 55.

3. La préparation selon la revendication 1, dans laquelle n est égal à 50 et le composé A est présent à hauteur d'environ 50 à environ 75 mg de la préparation.

4. La préparation selon la revendication 1, dans laquelle le composé A est présent à hauteur d'environ 30 à environ 75 mg de la préparation.

5. La préparation selon la revendication 4, dans laquelle dans le composé A, n est égal à 44, 45, 46, 47, 48, 49, 50, 51, 52, 53, 54 ou 55.

6. La préparation selon la revendication 1, dans laquelle le composé A est présent à hauteur d'environ 50 à environ 75 mg de la préparation.

7. La préparation selon la revendication 2, dans laquelle le composé A est présent à hauteur d'environ 50 à environ 75 mg de la préparation.

8. La préparation selon la revendication 2, dans laquelle le composé A est présent à hauteur d'environ 50 mg de la préparation.

9. La préparation selon la revendication 3, dans laquelle le composé A est présent à hauteur d'environ 30 à environ 75 mg de la préparation.

10. La préparation selon la revendication 3, dans laquelle le composé A est présent à hauteur d'environ 50 mg de la préparation.

11. La préparation selon la revendication 1, dans laquelle l'agent tampon est présent à hauteur d'environ 10 mM à environ 50 mM de la préparation.

12. La préparation selon la revendication 1, dans laquelle l'agent gonflant est du tréhalose amorphe et est présent à hauteur d'environ 75 à environ 95 mg de la préparation.

13. La préparation selon la revendication 1, dans laquelle l'agent gonflant est du dextrose et est présent à hauteur d'environ 30 mg à environ 75 mg de la préparation.

14. La préparation selon la revendication 13, dans laquelle le dextrose est présent à hauteur d'environ 40 à environ 60 mg de la préparation.

15. La préparation selon la revendication 1, dans laquelle l'agent gonflant est du mannitol et est présent à hauteur d'environ 25 mg à environ 75 mg de la préparation.

16. La préparation selon la revendication 15, dans laquelle le mannitol est présent à hauteur d'environ 30 à environ 60 mg de la préparation.

17. La préparation selon la revendication 1, dans laquelle l'agent gonflant est du saccharose et est présent à hauteur d'environ 70 mg à environ 110 mg de la préparation.

18. La préparation selon la revendication 17, dans laquelle le saccharose est présent à hauteur d'environ 75 à environ 100 mg de la préparation.

19. La préparation selon la revendication 1, dans laquelle l'agent gonflant est du lactose et est présent à hauteur d'environ 70 mg à environ 120 mg de la préparation.

20. La préparation selon la revendication 13, dans laquelle le lactose est présent à hauteur d'environ 90 à environ 110 mg de la préparation.

21. La préparation selon la revendication 1, dans laquelle l'agent tampon est de l'histidine et est présent à hauteur d'environ 10 mM à environ 100 mM de la préparation.

22. La préparation selon la revendication 21, dans laquelle l'histidine est présente à hauteur d'environ 10 mM à environ 50 mM de la préparation.

23. La préparation selon la revendication 1, dans laquelle l'agent gonflant est présent à hauteur d'environ 50 mg à environ 100 mg de la préparation.

24. Une préparation pharmaceutique lyophilisée comprenant environ 50 mg de l'ester 1-[2-(2-méthoxy-éthoxy)-éthoxycarbonyloxy]-éthylique de l'acide 4-{[(2R, 3S, 5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque (composé A) 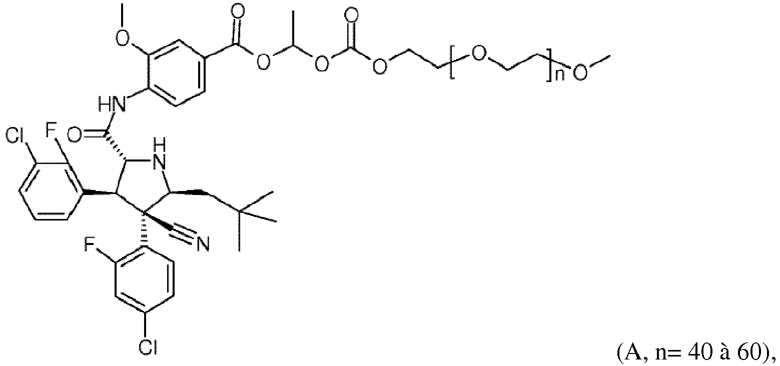 environ 3,1 mg d'histidine, environ 85 mg d'un tréhalose déshydraté et d'un adjuvant d'isotonicité, ladite préparation ayant un pH d'environ 5 à environ 7, dans un volume de reconstitution final d'environ 1 ml.

25. La préparation pharmaceutique lyophilisée selon la revendication 24, dans laquelle n est égal à 44, 45, 46, 47, 48, 49, 50, 51, 52, 53 ou 55.

26. La préparation pharmaceutique lyophilisée selon la revendication 25, dans laquelle n = 50.
